# EUROPEAN PATENT SPECIFICATION

(11) **EP 3 197 279 B1**
(45) Date of publication and mention of the grant of the patent: **09.12.2020**
(21) Application number: 15843942.2
(22) Date of filing: 23.09.2015
(51) Int. Cl.: A01N 59/16, A01P 1/00, A01P 3/00

(54) **ANTIMICROBIAL COMPOSITIONS AND METHODS**
ANTIMIKROBIELLE ZUSAMMENSETZUNGEN UND VERFAHREN
COMPOSITIONS ANTIMICROBIENNES ET PROCÉDÉS

(30) Priority: 23.09.2014 US 201462054152 P; 23.09.2014 US 201462054154 P; 22.09.2015 US 201514861243
(43) Date of publication of application: 02.08.2017
(73) Proprietor: Attostat, Inc., Salt Lake City, Utah 84121 (US)
(72) Inventor: NIEDERMEYER, William Harold, North Salt Lake, Utah 84054 (US)
(74) Representative: Schwarz & Partner Patentanwälte OG
(86) International application number: PCT/US2015/051639
(87) International publication number: WO 2016/049131

(56) References cited:
- EP-A1- 2 559 436
- EP-A2- 2 140 958
- WO-A1-2008/043396
- WO-A1-2009/025955
- WO-A1-2009/044146
- WO-A1-2009/091900
- WO-A1-2011/045627
- WO-A2-2006/026026
- WO-A2-2006/053225
- WO-A2-2006/062826
- WO-A2-2009/046081
- DE-A1-102005 044 360
- US-A1- 2008 050 448
- US-A1- 2009 028 947
- US-A1- 2009 148 484
- US-A1- 2010 040 655
- US-A1- 2010 040 655
- US-A1- 2010 180 413
- US-A1- 2010 180 413
- US-A1- 2010 272 770
- US-A1- 2013 001 833
- US-A1- 2013 152 823
- US-A1- 2013 334 104
- JINYUN LIU ET AL: "A novel coral-like porous SnO 2 hollow architecture: biomimetic swallowing growth mechanism and enhanced photovoltaic property for dye-sensitized solar cell application", CHEMICAL COMMUNICATIONS, vol. 46, no. 3, 1 January 2010 (2010-01-01), pages 472-474, XP55317057, ISSN: 1359-7345, DOI: 10.1039/B915650J
- LIU ET AL.: 'A novel coral-like porous Sn02 hollow architecture: biomimetic swallowing growth mechanism and enhanced photovoltaic property for dye-sensitized solar cell application.' CHEM. COMMUN. vol. 46, 2010, pages 472 - 474, XP055317057
- CHIEN ET AL.: 'Synthesis of nanoparticles: sunlight formation of gold nanodecahedra for ultra-sensitive lead-ion detection.' GREEN CHEM. vol. 13, May 2011, pages 1162 - 1166, XP055419810

## Description

### Field of the Invention

Disclosed herein are antimicrobial compositions and methods for making and using such compositions.

### 2. Relevant Technology

Diseases old and new caused by microbes such as bacteria, viruses, and fungi continue to plague humans, animals, and plants. In addition to infecting humans, sometimes as epidemics or pandemics, microbial diseases that affect animals and plants raised for food have and can continue to devastate food supplies. Food products are also prone to spoilage as a result of microbes, particularly bacteria and fungi.

Although modern science has yielded many new antimicrobial compositions, some, such as antibiotics, are not always effective because microbes can build up tolerance or immunity to such compositions. In addition, apart from natural immunity and acquired immunity from vaccinations, there are no compositions that can reliably target and selectively destroy viruses.

Besides specific compositions and drugs, there are non-specific ways to combat microbes and microbial diseases, such as burning, harsh chemicals, and partial or complete removal of diseased human, animal, or plant tissues. Because such measures are non-specific and often kill or severely affect the organism being treated, they are usually used as a last resort.

Among the deadliest human diseases are anthrax, caused by *Bacillus Anthracis;* ebola virsus disease; hemorrhagic fever, caused by Marburg virus; hantavirus; rabies; smallpox; Crimean-Congo hemorrhagic, fever caused by a tick-borne virus; avian influenza (bird flu); severe acute respiratory syndrome (SARS), caused by coronavirus; malaria, caused by mosquitos infected with Plasmodium parasites; typhoid fever caused by *Salmanella Typhi* bacterium; cholera, an acute intestinal infection caused by Vibrio cholera bacterium; yellow fever, a hemorrhagic fever transmitted by infected mosquitoes; acquired immune deficiency syndrome (AIDS); bubonic plague, caused by *Yersinia pestis* bacterium; and wound and flesh infections caused by many different bacteria, particularly those which are antibiotic-resistant.

There are also many plant diseases of many types that could threaten the world's food supply. An example of a potentially devastating plant disease is citrus greening disease, which affects citrus trees and for which there is no cure. Citrus greening disease is caused by motile bacteria, *Candidatus Liberibacter spp.* and transmitted by insects *(e.g.,* various types of psyllids). In the heyday of Florida citrus, around 1970, the number of acres with orange, grapefruit, and specialty fruit orchards surpassed 3642 square kilometres (900,000 acres). Today, it is reportedly slightly more than 2023 square kilometres (500,000 acres) as a result of citrus greening disease.

Botulism from consuming spoiled food is a relatively rare but serious and potentially fatal paralytic illness caused by a nerve toxin that is produced by the bacterium *Clostridium botulinum.*

Among the deadliest viral diseases is Ebola disease, with a mortality rate that is reportedly as high as 90%. Ebola virus disease (EVD), Ebola hemorrhagic fever (EHF), or simply Ebola is a disease of humans and other primates caused by an Ebola virus. Symptoms of the disease can start two days to three weeks after contracting the virus, with a fever, sore throat, muscle pain and headaches. Typically, vomiting, diarrhea and rash follow, along with decreased functioning of the liver and kidneys. Around this time, affected people may begin to bleed both within the body and externally.

EVD is caused by four of five viruses classified in the genus *Ebolavirus,* family *Filoviridae,* order *Mononegavirales.* The four disease-causing viruses are Bundibugyo virus (BDBV), Sudan virus (SUDV), Taï Forest virus (TAFV), and one called simply, Ebola virus (EBOV, formerly Zaire Ebola virus). Ebola virus is the sole member of the Zaire *ebolavirus* species, and the most dangerous of the known Ebola disease-causing viruses, as well as being responsible for the largest number of outbreaks. The fifth virus, Reston virus (RESTV), is not thought to be disease-causing in humans. The five Ebola viruses are closely related to Marburg viruses.

No specific treatment for EVD is yet available. Efforts to help those who are infected are supportive and include giving either oral rehydration therapy or intravenous fluids. EVD has a high risk of death, killing between 50% and 90% of those infected. EVD was first identified in Sudan (now South Sudan) and the Democratic Republic of the Congo. Efforts are under way to develop a vaccine; however, none yet exists.

Accordingly, there has been and remains a need to find compositions that can reliably target and preferentially kill or deactivate disease-causing microbes without killing or causing undue harm to the organism being treated (*e.g*., human, animal, or plant).

US 2009/148484 A1 describes a composite composition that combines Ag nanoparticles with inorganic clays.

US 2010/272770 A1 discloses a composition including silver nanoparticles and 20 to 80% by weight of a biocomponent of *Lactobacillus.*

WO 2009/091900 A1 describes processes making use of the free radicals in oils to reduce metal salts contained therein to form a dispersion of metal nanoparticles throughout the oil.

WO 2006/053225 A2 discloses protein metal nanoparticle complexes and their use as anti-virals.

US 2013/001833 A1 describes a laser ablation process for forming metal nanoparticles with a narrow size distribution.

Further state of the art is disclosed in US 2008/050448 A1, EP 2 140 958 A2, WO 2009/044146 A1, WO 2009/046081 A2, US 2010/180413 A1, Jinyun et al. (Chemical Communications, 2010, vol. 46, no. 3, pages 472-474), US 2010/040655 A1, US 2013/152823 A1, WO 2008/043396 A1, WO 2006/026026 A2, DE 10 2005 044360 A1, WO 2006/062826 A2, WO 2009/025955 A1, EP 2 559 436 A1, WO 2011/045627 A1 and US 2009/028947 A1.

### SUMMARY

Disclosed herein are antimicrobial compositions and methods for killing or deactivating a wide variety of harmful microbes, such as viruses, bacteria, and fungi, which can infect humans, animals, plants, or food supplies. Also disclosed are methods for making antimicrobial compositions. Unexpectedly, it has now been found that by selecting metal nanoparticles of a particular size and particle size distribution it is possible to target and preferentially kill or deactivate a specific type of microbe.

The present invention provides an antimicrobial composition for use in the treatment of an infection or a disease in a living human or animal caused by a microbe according to claim 1 and the claims dependent therefrom, and a method for deactivating or preferentially killing a microbe according to claim 5 and the claims dependent therefrom.

An antimicrobial composition according to the invention comprises (1) a carrier and (2) a plurality of metal nanoparticles having a particle size and a particle size distribution selected so as to selectively deactivate or preferentially kill one of a virus, bacterium, or fungus.

Anti-viral compositions include metal nanoparticles having a particle size of 2 nm to 6.5 nm, such as 3 nm to 6 nm. Within these size ranges it is possible to select "designer anti-viral particles" of specific size that are particularly effective in targeting a specific virus.

Anti-bacterial compositions include metal nanoparticles having a particle size of 7 nm to 12 nm, such as 8 nm to 10 nm. Within these size ranges it is possible to select "designer anti-bacterial particles" of specific size that are particularly effective in targeting a specific bacterium.

Anti-fungal compositions include metal nanoparticles having a particle size of 10 nm to 18 nm, such as 11 nm to 16 nm, or 12 nm to 15 nm. Within these size ranges it is possible to select "designer anti-fungal particles" of specific size that are particularly effective in targeting a specific fungus.

A method of deactivating or preferentially killing a microbe according to the invention comprises: (1) applying an antimicrobial composition as defined in claim 5 onto or into a substrate containing the microbe, and (2) the antimicrobial composition deactivating or killing the microbes. The substrate is a non-living object.

These and other advantages and features of the invention will be set forth in part in the description which follows, and in part will become apparent to those skilled in the art upon examination of the following or may be learned by practice of the invention.

### BRIEF DESCRIPTION OF THE DRAWINGS

FIG. 1 is a transmission electron microscope image (TEM) of exemplary spherical-shaped metal nanoparticles having substantially uniform size and narrow particle size distribution for use in making antimicrobial compositions;
FIG. 2 schematically illustrates a microbe after having absorbed spherical-shaped metal nanoparticles from a substrate;
FIG. 3 schematically illustrates a microbe protein with disulfide bonds being catalytically denatured by an adjacent spherical-shaped nanoparticle; and
FIG. 4 schematically illustrates a mammalian protein with disulfide bonds that are shielded so as to resist being catalytically denatured by an adjacent spherical-shaped nanoparticle.

### DETAILED DESCRIPTION OF THE PREFERRED EMBODIMENTS

Disclosed herein are antimicrobial compositions and methods for killing or deactivating microbes, such as viruses, bacteria, or fungi. The metal nanoparticles are dispersed within or contained on or within a carrier that can be applied onto or into a substrate containing a microbe. The carrier can be a liquid, gel or solid. The antimicrobial compositions is formulated to selectively and preferentially kill one or more specific microbes.

The antimicrobial compositions comprise a carrier and a plurality of metal nanoparticles having a particle size and a particle size distribution selected so as to selectively deactivate or preferentially kill one of a virus, a bacterium, or a fungus. Anti-viral compositions comprise metal nanoparticles having a particle size of 2 nm to 6.5 nm, such as 3 nm to 6 nm. Anti-bacterial compositions include metal nanoparticles having a particle size of 7 nm to 12 nm, such as 8 nm to 10 nm. Anti-fungal compositions include metal nanoparticles having a particle size of 10 nm to 18 nm, such as 11 nm to 16 nm, or 12 nm to 15 nm. Within any of the foregoing size ranges, it is possible to select "designer antimicrobial particles" of specific size that are particularly effective in targeting a specific microbe.

The ability to select and use microbe-specific nanoparticles provides a number of benefits. In the case where only certain nanoparticle sizes are effective in killing a particular microbe or class of microbes, providing metal nanoparticles within a narrow particle size distribution of the correct particle size maximizes the proportion of nanoparticles that are effective in killing the target microbe and minimizes the proportion of nanoparticles that are less effective, or ineffective, in killing the target microbe. This, in turn, greatly reduces the overall amount or concentration of nanoparticles required to provide a desired kill- or deactivation rate of a targeted microbe. Eliminating improperly-sized nanoparticles also reduces the tendency of the composition to kill or harm non-targeted microbes or other cells, such as healthy mammalian or human cells. In this way, highly specific antimicrobial compositions can better target a harmful microbe while being less harmful or even non-toxic to humans, animals, and plants.

### Nanoparticle Configurations

The plurality of metal nanoparticles consists of nonionic, ground state spherical-shaped metal nanoparticles.

The nonionic metal nanoparticles useful for making antimicrobial compositions consist of spherical nanoparticles, preferably spherical-shaped metal nanoparticles having a solid core. The term "spherical-shaped metal nanoparticles" refers to nanoparticles that are made from one or more nonionic, ground state metals, having only internal bond angles and no external edges or bond angles. In this way, the spherical nanoparticles are highly resistant to ionization, highly stable, and highly resistance to agglomeration. Such nanoparticles can exhibit a high ξ-potential, which permits the spherical nanoparticles to remain dispersed within a polar solvent without a surfactant, which is a surprising and expected result.

In general, spherical-shaped metal nanoparticles can have a diameter of 40 nm or less, 35 nm or less, 30 nm or less, 25 nm or less, 20 nm or less, 15 nm or less, 10 nm or less, 7.5 nm or less, or 5 nm or less. Nevertheless, selecting spherical-shaped nanoparticles of specific particle size and narrow particle size distribution is particularly useful for selectively deactivating or preferentially killing a particular type of microbe, such as a virus, a bacterium, or a fungus.

The spherical-shaped nanoparticles have a particle size distribution such that at least 99% of the nanoparticles have a diameter within 30% of the mean diameter of the nanoparticles, such as within 20% of the mean diameter, or within 10% of the mean diameter. In some embodiments, spherical-shaped nanoparticles can have a ξ-potential of at least 10 mV, preferably at least 15 mV, more preferably at least 20 mV, even more preferably at least 25 mV, and most preferably at least 30 mV.

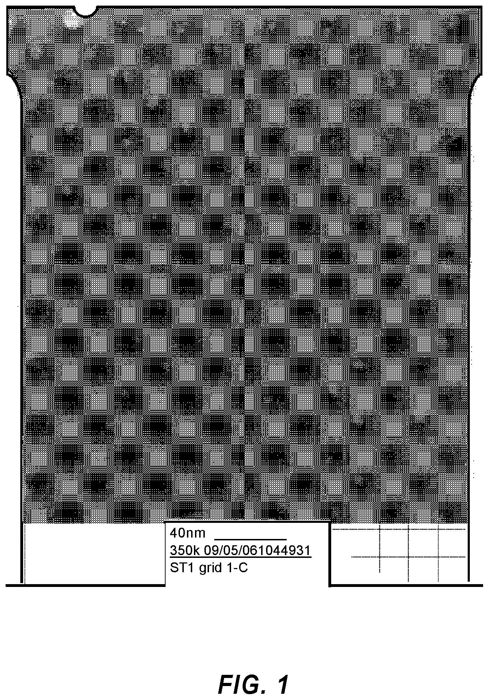

Examples of methods and systems for manufacturing spherical-shaped nanoparticles are disclosed in U.S. Pat. Pub. No. 2013/0001833 to William Niedermeyer ("Niedermeyer Publication"). FIG. 1 is a transmission electron microscope image (TEM) of exemplary spherical-shaped nanoparticles made using the methods and systems of the Niedermeyer Publication. The illustrated nanoparticles are spherical-shaped silver (Ag) nanoparticles of substantially uniform size, with a mean diameter of about 10 nm and a narrow particle size distribution. In some embodiments, spherical-shaped nanoparticles can have a solid core rather than being hollow, as is the case with conventional metal nanoparticles, which are usually formed on the surfaces of non-metallic seed nanoparticles (*e.g.,* silica), which are thereafter removed to yield hollow nanospheres.

The metal nanoparticles may additionally comprise any desired metal, mixture of metals, or metal alloy, including at least one of gold, platinum, palladium, osmium, ruthenium, rhodium, rhenium, molybdenum, copper, iron, nickel, tin, beryllium, cobalt, antimony, chromium, manganese, zirconium, zinc, tungsten, titanium, vanadium, lanthanum, cerium, heterogeneous mixtures thereof, or alloys thereof.

The antimicrobial metal nanoparticles will comprise at least silver. In the case of larger metal nanoparticles for use in killing bacteria and fungi, metal nanoparticles may primarily or exclusively comprise silver. On the other hand, in some embodiments, a gold-silver alloy provides the particle stabilizing activity of gold and potentially higher anti-viral activity of silver.

### Antimicrobial Activity

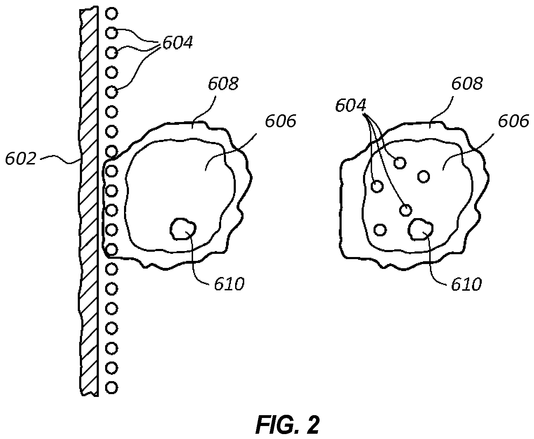

FIG. 2 schematically illustrates a microbe 608 having absorbed spherical-shaped nanoparticles 604 from a solid substrate 602, such as by active absorption or other transport mechanism. Alternatively, spherical-shaped nanoparticles 604 can be provided in a composition (not shown), such as in a liquid or gel carrier. The nanoparticles 604 can freely move throughout the interior 606 of microbe 608 and come into contact with one or more vital proteins or enzymes 610 that, if denatured, will kill or disable the microbe.

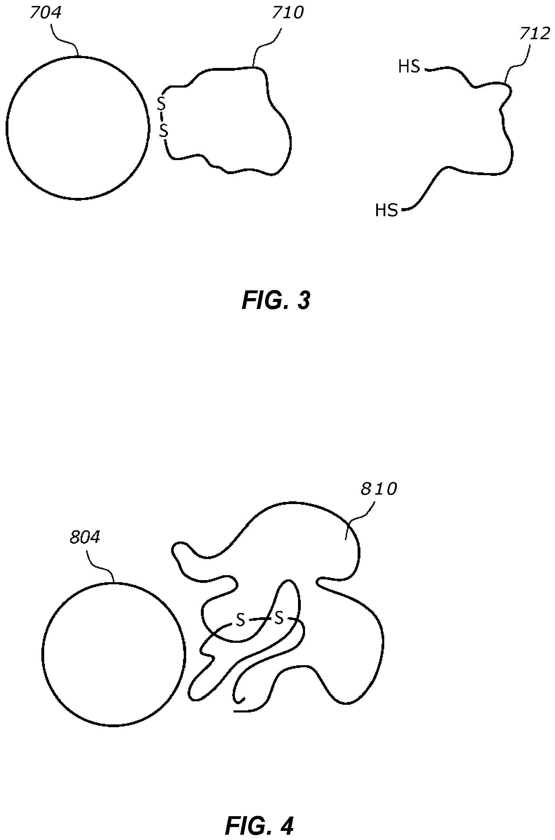

One way that nanoparticles may kill or denature a microbe is by catalyzing the cleavage of disulfide (S-S) bonds within a vital protein or enzyme. FIG. 3 schematically illustrates a microbe protein or enzyme 710 with disulfide bonds being catalytically denatured by an adjacent spherical-shaped nanoparticle 704 to yield denatured protein or enzyme 712. In the case of bacteria or fungi, the cleavage of disulfide bonds and/or cleavage of other chemical bonds of vital proteins or enzymes may occur within the cell interior and thereby killing the microbe in this manner. Such catalytic cleavage of disulfide (S-S) bonds is facilitated by the generally simple protein structures of microbes, in which many vital disulfide bonds are on exposed and readily cleaved by catalysis.

Another mechanism by which metal (e.g., silver) nanoparticles can kill microbes is through the production of active oxygen species, such as peroxides, which can oxidatively cleave protein bonds, including but not limited to amide bonds.

In the case of viruses, spherical-shaped metal nanoparticles can alternatively deactivate viruses by attaching to glycoproteins and/or catalyzing protein denaturing reactions in the protein coat so that the virus is no longer able to attach to a host cell and/or inject genetic material into the host cell. Because very small nanoparticles can pass through a virus, denaturing of the protein coat may occur within the interior of the virus. A virus that is rendered unable to attach to a host cell and/or inject genetic material into the host cell is essentially inactive and no longer pathogenic.

Notwithstanding the lethal nature of nonionic metal nanoparticles relative to microbes, they can be relatively harmless to humans, mammals, and healthy mammalian cells, which contain much more complex protein structures compared to simple microbes in which most or all vital disulfide bonds are shielded by other, more stable regions of the protein. FIG. 4 schematically illustrates a mammalian protein 810 with disulfide (S-S) bonds that are shielded so as to resist being catalytically denatured by an adjacent spherical-shaped nanoparticle 804. In many cases the nonionic nanoparticles do not interact with or attach to human or mammalian cells, remain in and follow fluid flow, do not cross barriers, remain in the vascular system, and can be quickly and safely expelled through the urine without damaging kidneys or other cells.

In the particular case of silver (Ag) nanoparticles, the interaction of the silver (Ag) nanoparticle(s) within a microbe has been demonstrated to be particularly lethal without the need to rely on the production of silver ions (Ag⁺) to provide the desired antimicrobial effects, as is typically the case with conventional colloidal silver compositions. The ability of silver (Ag) nanoparticles to provide effective microbial control without any significant release of toxic silver ions (Ag⁺) into the surrounding environment is a substantial advancement in the art. In addition, obtaining very small nanoparticles as described herein made of a mixture or alloy of gold and silver atoms, wherein the gold has greater bonding ability and silver greater anti-microbial activity, is a substantial advancement in the art.

As discussed herein, the size of the nanoparticles is selected to target and selectively kill specific types of microbes, Using very small nonionic metal nanoparticles as described herein, permits the nanoparticles to more easily penetrate into and promote denaturing reactions within the protein coat, such as cleavage of disulfide (S-S) bonds in capsid proteins of the virus. Additionally, or alternatively, such nanoparticles can also bind to glycoproteins of the protein coat. Either or both mechanisms can prevent a virus from attaching to a host cell and introducing its genetic material therein.

### Microbe-Specific Nanoparticles

Spherical-shaped nanoparticles designed to selectively and preferentially deactivate viruses have a diameter of 2 nm to 6.5 nm, or 3 nm to 6 nm. Within these size ranges it is possible to select "designer anti-viral particles" of specific size that are particularly effective in targeting a specific virus.

Spherical-shaped nanoparticles designed to selectively and preferentially deactivate bacteria have a diameter of 7 nm to 12 nm, or 8 nm to 10 nm. Within these size ranges it is possible to select "designer anti-bacterial particles" of specific size that are particularly effective in targeting a specific bacterium.

Spherical-shaped nanoparticles designed to selectively and preferentially deactivate fungi have a diameter of 10 nm to 18 nm, 11 nm to 16 nm, or 12 nm to 15 nm. Within these size ranges it is possible to select "designer anti-fungal particles" of specific size that are particularly effective in targeting a specific fungus.

The antimicrobial metal nanoparticles will comprise at least silver . In the case of larger metal nanoparticles for use in killing bacteria and fungi, the metal nanoparticles may primarily or exclusively comprise silver. On the other hand, a gold-silver alloy provides the particle stabilizing activity of gold and the higher microbe killing activity of silver.

As discussed herein, the size of the nanoparticles is selected to target and selectively kill specific types of microbes.

### Carriers

A nanoparticle composition includes a carrier for delivering the metal nanoparticles onto or into a living or non-living substrate. The carrier can be a liquid, gel, or solid. Some carriers may be more suitable than others depending on the living or non-living substrate being treated. For example, the solubility characteristics of the carrier can be selected to maximize or otherwise provide a desired diffusion throughout a treated organism part and/or another portion of the organism in contact with the treated organism part.

Examples of compounds that can be utilized for topical applications and can be used as carriers include, but are not limited to, water, alcohols, ketones, esters, citrus oils, essential oils, vegetable and other plant and natural oils, triglycerides, ethers, organic solvents, methanol, ethanol, isopropyl alcohol, other alcohols, glycols, glycerin, polyols, 1,3-propandiol, petroleum jelly, waxes, polymers, polymerizable materials, and surfactants.

In one embodiment, the carrier is a cream or lotion including a glycerin and/or stearic acid cream base optionally containing oils such as coconut oil, olive oil, grape seed oil, shea butter, mango butter, and/or vitamin E oil along with an emulsifying wax.

In other embodiments the carrier is a water or combined water and alcohol solution which itself contains a micro to millimolar concentration of a separate stabilizing agent dissolved into the carrier so as to maintain the nanoparticles within the overall composition.

Exemplary carriers for nasal or pulmonary aerosol or inhalation administration include solutions in saline which can contain, for example, benzyl alcohol or other suitable preservatives, absorption promoters to enhance bioavailability, and/or other solubilizing or wetting or dispersing agents, such as glycerin, a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycethanol), a condensation product of ethylene oxide with a partial ester derived from a fatty acid and a hexitol anhydride (e.g., polyoxyethylene sorbitan monooleate); polysaccharides and polysaccharide-like compounds (e.g. dextran sulfate); and glycoaminoglycans and glycosaminoglycan-like compounds (e.g., hyaluronic acid), for example. In some embodiments, the nanoparticles and additional stabilizing agents and/or carriers are formulated as dry powders (e.g., powders useful for administering with dry powder inhalers).

Exemplary aerosols useful for nasal and/or inhalation administration include a vaporizable propellant, such as low molecular weight hydrofluorocarbons or hydrocarbons that are liquid when constrained in a suitable container and are biocompatible and nonirritating. Ingredients such as water, alcohol, propylene glycol, and polyethylene glycols can be additionally included. Other embodiments, also useful for nasal and/or inhalation administration, are provided as sprays (e.g., omitting an aerosol propellant). Such spray formulation may be provided as a solution, suspension, or emulsion capable of forming a fine mist for administration, and in some embodiments, may include saline and/or be isotonic.

Exemplary injectable solutions include an aqueous emulsion or oleaginous suspension or saline solution (e.g., isotonic, hypotonic, or hypertonic, optionally including dextrose and/or other electrolytes or additives). Such compositions can also include suitable dispersing or wetting agents. The sterile injectable preparation may also be formed in a non-toxic parenterally acceptable diluent or solvent, such as a solution in 1,2-propanediol (propylene glycol). Additional examples include solutions or suspensions which can contain, for example, suitable non-toxic diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Gels known in the art can be used as carriers, such as gels containing one or more of the foregoing liquid components together with known gelling agents. Gel compositions can more easily adhere to a living or non-living substrate being treated. An exemplary gel carrier can include mineral oil gelled with polyethylene.

Solid carriers can be used for different reasons, such as to elute nanoparticles into an organism over time. Examples of solid carriers include, but are not limited to, polymers, rubbers, elastomers, foams, and gums. Depending on the characteristics of the organism to be treated and the desired rate of elution, one of skill in the art can select an appropriate solid carrier material.

The antimicrobial composition is formulated so that the metal nanoparticles are included in a concentration so that a measured quantity of the nanoparticle composition, when applied onto or into an organism or organism part, will provide a predetermined concentration or quantity of metal nanoparticles. The nanoparticle composition can have a higher concentration of nanoparticles that become diluted when mixed with other liquids applied to or naturally contained within the organism or organism part. The antimicrobial composition contains 10 ppb (parts per billion) to 100 ppm (parts per million) by weight of the antimicrobial composition, or 15 ppb to 90 ppm, or 100 ppb to 75 ppm, or 500 ppb to 60 ppm of metal nanoparticles by weight, or 1 ppm to 50 ppm, or 2 ppm to 25 ppm, or 3 ppm to 20 ppm metal nanoparticles by weight of the antimicrobial composition.

In some embodiments, the antimicrobial composition can also include one or more optional components or adjuvents to provide desired properties, including, but not limited to food, vitamins, minerals, antimicrobial agents, electrolytes, moisturizers, emollients, antiseptics, and/or plant extracts.

In some embodiments, the carrier may also function as, or may include, a stabilizing agent. For example, in some embodiments it may be desirable to have different specifically sized nanoparticles within the same solution to take advantage of each of the different properties and effects of the different particles. However, when differently sized particles are mixed into a single solution, the overall long-term stability of these particles within that single solution may be substantially diminished as a result of unequal forces exerted on the various particles causing eventual agglomeration of the particles. This phenomenon may become even more pronounced when that solution is either heated or cooled significantly above or below standard room temperature conditions.

Examples of stabilizing agents include alcohols (*e.g*., ethanol, propanol, butanol, etc.), polyphenols (*e.g*., arjuna bark extract, grape seed extract, etc.), mono-glycerides, diglycerides, or triglycerides (*e.g.,* grape seed oil, coconut oil, and the like), oils (*e.g.,* lavender), other terpenes, amine compounds (*e.g*., mono-, di-, or tri-ethanol amine), carbohydrates (*e.g*., sucrose, fructose), liposomes, creams, other emulsions, and other polymers.

In some embodiments, stabilizing agents are dissolved within a separate carrier in the micro- to milli- molar concentration range with the upper range limitation typically being constrained not by efficacy but by product cost.

These various stabilizing agents have the capacity to hold the at least two differently sized and/or shaped nanoparticles in suspension and deliver these nanoparticles into the treatment area of a person or animal without so powerfully retaining the nanoparticles so as to diminish the antimicrobial properties of the nanoparticles.

### Treatment Methods

A method of killing or deactivating a microbe according to the invention comprises: (1) applying an antimicrobial composition as defined in claim 5, onto or into a non-living substrate containing microbes; and (2) the antimicrobial composition killing or denaturing the microbes. The substrate is a non-living object that has come into contact, or is at risk of coming into contact, with a disease causing or otherwise unwanted microbe, such as clothing, bedding, wound dressings, medical devices or implants, food, intravenously injectable liquids, farm equipment, animal feeding devices, watering troughs, and the like.

In some embodiments, metal nanoparticles kill bacteria or fungi by entering the cell and catalyzing protein denaturing reactions. In some embodiments, metal nanoparticles "deactivate" viruses by attaching to glycoproteins and/or catalyzing protein denaturing reactions in the protein coat. Examples of protein denaturing reactions include reactions involving one or more of disulfide (S-S) bond cleavage and formation of active oxygen species that attack amide or other bonds.

In some embodiments, a method of deactivating a virus comprises: (1) applying an anti-viral composition comprising metal nanoparticles having the selected particle size onto or into a non-living substrate containing a virus, and (2) the anti-viral composition deactivating the virus.

In some embodiments, metal nanoparticles deactivate viruses by attaching to glycoproteins and/or catalyzing protein denaturing reactions in the protein coat. Examples of protein denaturing reactions include reactions involving one or more of disulfide (S-S) bond cleavage and formation of active oxygen species that attack amide or other bonds.

In some embodiments, the method may comprise applying the anti-viral composition to one or more of medical equipment, clothing, bandages, waste receptacles, syringes, syringe needles, inhalation equipment, or implants.

A method of killing a bacterium comprises: (1) applying an anti-bacterial composition comprising metal nanoparticles having the selected particle size onto or into a non-living substrate containing a bacterium, and (2) the anti-bacterial composition killing the bacterium.

A method of killing a fungus comprises: (1) applying an anti-fungal composition comprising metal nanoparticles having the selected particle size onto or into a non-living substrate containing a fungus, and (2) the anti-fungal composition killing the fungus.

## Claims

1. An antimicrobial composition for use in the treatment of an infection or a disease in a living human or animal caused by a microbe, wherein the microbe is deactivated or preferentially killed, the microbe being one of a virus, a bacterium, or a fungus, comprising:
a carrier, such as a liquid, gel, or solid; and
a plurality of metal nanoparticles having a particle size and a particle size distribution selected so as to selectively deactivate or preferentially kill one of a virus, a bacterium, or a fungus, **characterized by**:
the antimicrobial composition containing 10 ppb to 100 ppm of the metal nanoparticles by weight of the antimicrobial composition; and
the plurality of metal nanoparticles consisting of nonionic, ground state, spherical-shaped metal nanoparticles having a mean diameter wherein at least 99% of the spherical-shaped nanoparticles have a diameter within 30% of the mean diameter, such as within 20% of the mean diameter, or within 10% of the mean diameter, and wherein at least a portion of the metal nanoparticles comprise silver nanoparticles, wherein
when the microbe is a virus, the metal nanoparticles have a particle size of 2 nm to 6.5 nm, such as 3 nm to 6 nm, or
when the microbe is a bacterium, the metal nanoparticles have a particle size of 7 nm to 12 nm, such as 8 nm to 10 nm, or
when the microbe is a fungus, the metal nanoparticles have a particle size of 10 nm to 18 nm, such as 11 nm to 16 nm, or 12 nm to 15 nm.

2. An antimicrobial composition for the use as in claim 1, wherein the spherical-shaped nanoparticles have a ξ-potential of at least 10 mV, or at least 15 mV, or at least 20 mV, or at least 25 mV, or at least 30 mV.

3. An antimicrobial composition for the use as in claim 1 or 2, wherein the carrier is a liquid in which the metal nanoparticles are colloidally dispersed, such as wherein the liquid is at least one of a non-polar liquid, an organic solvent, a polar liquid, or an aqueous liquid.

4. An antimicrobial composition for the use as in any one of claims 1 to 3, wherein the metal nanoparticles comprise at least one additional metal selected from the group consisting of gold, platinum, palladium, rhodium, osmium, ruthenium, rhenium, molybdenum, copper, iron, nickel, tin, beryllium, cobalt, antimony, chromium, manganese, zirconium, zinc, tungsten, titanium, vanadium, lanthanum, cerium, heterogeneous mixtures thereof, and alloys thereof.

5. A method of deactivating or preferentially killing a microbe, the microbe being one of a virus, a bacterium, or a fungus, comprising:
applying an antimicrobial composition onto or into a non-living substrate containing the microbe, wherein the antimicrobial composition comprises:
a carrier, such as a liquid, gel, or solid; and
a plurality of metal nanoparticles having a particle size and a particle size distribution selected so as to selectively deactivate or preferentially kill one of a virus, a bacterium, or a fungus, **characterized by**:
the antimicrobial composition containing 10 ppb to 100 ppm of the metal nanoparticles by weight of the antimicrobial composition; and
the plurality of metal nanoparticles consisting of nonionic, ground state, spherical-shaped metal nanoparticles having a mean diameter wherein at least 99% of the spherical-shaped metal nanoparticles have a diameter within 30% of the mean diameter, such as within 20% of the mean diameter, or within 10% of the mean diameter, and
wherein at least a portion of the metal nanoparticles comprise silver nanoparticles,
wherein the particle size of the metal nanoparticles is selected based on whether the microbe is a virus, a bacterium, or a fungus;
wherein
when the microbe is a virus, the metal nanoparticles have a particle size of 2 nm to 6.5 nm, such as 3 nm to 6 nm, or
when the microbe is a bacterium, the metal nanoparticles have a particle size of 7 nm to 12 nm, such as 8 nm to 10 nm, or
when the microbe is a fungus, the metal nanoparticles have a particle size of 10 nm to 18 nm, such as 11 nm to 16 nm, or 12 nm to 15 nm, and
the antimicrobial composition deactivating or killing the microbe.

6. A method according to claim 5, wherein the antimicrobial composition is **characterized by** the features of one or more of claims 2 to 4.

## Patentansprüche

1. Antimikrobielle Zusammensetzung zur Verwendung bei der Behandlung einer Infektion oder einer Krankheit bei einem lebenden Menschen oder Tier, die durch eine Mikrobe verursacht ist, wobei die Mikrobe deaktiviert oder bevorzugt abgetötet wird, wobei die Mikrobe entweder ein Virus, ein Bakterium oder ein Pilz ist, umfassend:
einen Träger, wie z.B. eine Flüssigkeit, ein Gel oder einen Feststoff; und
eine Mehrzahl von Metallnanopartikeln mit einer Partikelgröße und einer Partikelgrößenverteilung, die so ausgewählt sind, dass sie entweder ein Virus, ein Bakterium oder einen Pilz gezielt deaktivieren oder bevorzugt abtöten, **dadurch gekennzeichnet, dass**:
die antimikrobielle Zusammensetzung 10 ppb bis 100 ppm Metallnanopartikel, bezogen auf das Gewicht der antimikrobiellen Zusammensetzung, enthält; und
die Mehrzahl von Metallnanopartikeln aus nichtionischen kugelförmigen Metallnanopartikeln im Grundzustand besteht, die einen mittleren Durchmesser aufweisen, wobei mindestens 99% der kugelförmigen Nanopartikel einen Durchmesser innerhalb von 30% des mittleren Durchmessers, wie z.B. innerhalb von 20% des mittleren Durchmessers oder innerhalb von 10% des mittleren Durchmessers, aufweisen und wobei zumindest ein Teil der Metallnanopartikel Silbernanopartikel umfasst, wobei
die Metallnanopartikel eine Partikelgröße von 2 nm bis 6,5 nm, wie z.B. von 3 nm bis 6 nm, aufweisen, wenn die Mikrobe ein Virus ist, oder
die Metallnanopartikel eine Partikelgröße von 7 nm bis 12 nm, wie z.B. von 8 nm bis 10 nm, aufweisen, wenn die Mikrobe ein Bakterium ist, oder
die Metallnanopartikel eine Partikelgröße von 10 nm bis 18 nm, wie z.B. von 11 nm bis 16 nm oder von 12 nm bis 15 nm, aufweisen, wenn die Mikrobe ein Pilz ist.

2. Antimikrobielle Zusammensetzung zur Verwendung wie in Anspruch 1, wobei die kugelförmigen Nanopartikel ein ξ-Potential von mindestens 10 mV oder mindestens 15 mV oder mindestens 20 mV oder mindestens 25 mV oder mindestens 30 mV aufweisen.

3. Antimikrobielle Zusammensetzung zur Verwendung wie in Anspruch 1 oder 2, wobei der Träger eine Flüssigkeit ist, in der die Metallnanopartikel kolloidal verteilt sind, z.B. wobei die Flüssigkeit zumindest eines von einer unpolaren Flüssigkeit, einem organischen Lösungsmittel, einer polaren Flüssigkeit oder einer wässrigen Flüssigkeit ist.

4. Antimikrobielle Zusammensetzung zur Verwendung wie in einem der Ansprüche 1 bis 3, wobei die Metallnanopartikel mindestens ein zusätzliches Metall umfassen, das ausgewählt ist aus der Gruppe, bestehend aus Gold, Platin, Palladium, Rhodium, Osmium, Ruthenium, Rhenium, Molybdän, Kupfer, Eisen, Nickel, Zinn, Beryllium, Kobalt, Antimon, Chrom, Mangan, Zirkon, Zink, Wolfram, Titan, Vanadium, Lanthan, Cer, heterogenen Gemischen davon und Legierungen davon.

5. Verfahren zum Deaktivieren oder bevorzugt Abtöten einer Mikrobe, wobei die Mikrobe entweder ein Virus, ein Bakterium oder ein Pilz ist, umfassend:
das Applizieren einer antimikrobiellen Zusammensetzung auf oder in ein nichtlebendes Substrat, das die Mikrobe enthält, wobei die antimikrobielle Zusammensetzung Folgendes umfasst:
einen Träger, wie z.B. eine Flüssigkeit, ein Gel oder einen Feststoff; und
eine Mehrzahl von Metallnanopartikeln mit einer Partikelgröße und einer Partikelgrößenverteilung, die so ausgewählt sind, dass sie entweder ein Virus, ein Bakterium oder einen Pilz gezielt deaktivieren oder bevorzugt abtöten, **dadurch gekennzeichnet, dass**:
die antimikrobielle Zusammensetzung 10 ppb bis 100 ppm Metallnanopartikel, bezogen auf das Gewicht der antimikrobiellen Zusammensetzung, enthält; und
die Mehrzahl von Metallnanopartikeln aus nichtionischen kugelförmigen Metallnanopartikeln im Grundzustand besteht, die einen mittleren Durchmesser aufweisen, wobei mindestens 99% der kugelförmigen Metallnanopartikel einen Durchmesser innerhalb von 30% des mittleren Durchmessers, wie z.B. innerhalb von 20% des mittleren Durchmessers oder innerhalb von 10% des mittleren Durchmessers, aufweisen und
wobei zumindest ein Teil der Metallnanopartikel Silbernanopartikel umfasst,
wobei die Partikelgröße der Metallnanopartikel darauf basierend ausgewählt wird, ob die Mikrobe ein Virus, ein Bakterium oder ein Pilz ist;
wobei die Metallnanopartikel eine Partikelgröße von 2 nm bis 6,5 nm, wie z.B. von 3 nm bis 6 nm, aufweisen, wenn die Mikrobe ein Virus ist, oder
die Metallnanopartikel eine Partikelgröße von 7 nm bis 12 nm, wie z.B. von 8 nm bis 10 nm, aufweisen, wenn die Mikrobe ein Bakterium ist, oder
die Metallnanopartikel eine Partikelgröße von 10 nm bis 18 nm, wie z.B. von 11 nm bis 16 nm oder von 12 nm bis 15 nm, aufweisen, wenn die Mikrobe ein Pilz ist, und
wobei die antimikrobielle Zusammensetzung die Mikrobe deaktiviert oder abtötet.

6. Verfahren gemäß Anspruch 5, wobei die antimikrobielle Zusammensetzung durch die Merkmale eines oder mehrerer der Ansprüche 2 bis 4 gekennzeichnet ist.

## Revendications

1. Composition antimicrobienne à utiliser dans le traitement d'une infection ou d'une maladie chez un humain ou un animal vivant causée par un microbe, dans lequel le microbe est désactivé ou de préférence tué, le microbe étant l'un d'un virus, d'une bactérie ou d'un champignon, comprenant :
un support, tel qu'un liquide, un gel ou un solide ; et
une pluralité de nanoparticules métalliques ayant une taille de particule et une distribution de taille de particule choisies de manière à désactiver sélectivement ou de préférence tuer l'un d'un virus, d'une bactérie ou d'un champignon, **caractérisée par** :
la composition antimicrobienne contenant 10 ppb à 100 ppm des nanoparticules métalliques en poids de la composition antimicrobienne ; et
la pluralité de nanoparticules métalliques consistant en des nanoparticules métalliques non ioniques, à l'état fondamental, de forme sphérique, ayant un diamètre moyen, dans laquelle au moins 99 % des nanoparticules de forme sphérique ont un diamètre de 30 % ou moins du diamètre moyen, par exemple de 20 % ou moins du diamètre moyen, ou de 10 % ou moins du diamètre moyen, et dans lequel au moins une partie des nanoparticules métalliques comprend des nanoparticules d'argent, dans laquelle
lorsque le microbe est un virus, les nanoparticules métalliques ont une taille de particule de 2 nm à 6,5 nm, comme de 3 nm à 6 nm, ou
lorsque le microbe est une bactérie, les nanoparticules métalliques ont une taille de particule de 7 nm à 12 nm, comme de 8 nm à 10 nm, ou
lorsque le microbe est un champignon, les nanoparticules métalliques ont une taille de particule de 10 nm à 18 nm, comme de 11 nm à 16 nm, ou de 12 nm à 15 nm.

2. Composition antimicrobienne pour l'utilisation selon la revendication 1, dans laquelle les nanoparticules de forme sphérique ont un potentiel ζ d'au moins 10 mV, ou d'au moins 15 mV, ou d'au moins 20 mV, ou d'au moins 25 mV, ou d'au moins 30 mV.

3. Composition antimicrobienne pour l'utilisation selon la revendication 1 ou 2, dans laquelle le support est un liquide dans lequel les nanoparticules métalliques sont dispersées de manière colloïdale, comme dans laquelle le liquide est au moins un parmi un liquide non polaire, un solvant organique, un liquide polaire ou un liquide aqueux.

4. Composition antimicrobienne pour l'utilisation selon l'une quelconque des revendications 1 à 3, dans laquelle les nanoparticules métalliques comprennent au moins un métal supplémentaire choisi dans le groupe comprenant l'or, le platine, le palladium, le rhodium, l'osmium, le ruthénium, le rhénium, le molybdène, le cuivre, le fer, le nickel, l'étain, le béryllium, le cobalt, l'antimoine, le chrome, le manganèse, le zirconium, le zinc, le tungstène, le titane, le vanadium, le lanthane, le cérium, des mélanges hétérogènes de ceux-ci, et des alliages de ceux-ci.

5. Procédé pour désactiver ou de préférence tuer un microbe, le microbe étant l'un d'un virus, d'une bactérie ou d'un champignon, comprenant les étapes consistant à :
Appliquer une composition antimicrobienne sur ou dans un substrat non vivant contenant le microbe, dans lequel la composition antimicrobienne comprend :
un support, tel qu'un liquide, un gel ou un solide ; et
une pluralité de nanoparticules métalliques ayant une taille de particule et une distribution de taille de particule choisies de manière à désactiver sélectivement ou de préférence tuer l'un d'un virus, d'une bactérie ou d'un champignon, **caractérisé par** :
la composition antimicrobienne contenant 10 ppb à 100 ppm des nanoparticules métalliques en poids de la composition antimicrobienne ; et
la pluralité de nanoparticules métalliques constituées de nanoparticules métalliques non ioniques, à l'état fondamental, de forme sphérique, ayant un diamètre moyen, dans lequel au moins 99 % des nanoparticules de forme sphérique ont un diamètre de 30 % ou moins du diamètre moyen, comme de 20 % ou moins du diamètre moyen, ou de 10 % ou moins du diamètre moyen, et
dans lequel au moins une partie des nanoparticules métalliques comprend des nanoparticules d'argent,
dans lequel la taille de particule des nanoparticules métalliques est choisie en fonction du fait que le microbe est un virus, une bactérie ou un champignon ;
dans lequel
lorsque le microbe est un virus, les nanoparticules métalliques ont une taille de particule de 2 nm à 6,5 nm, comme de 3 nm à 6 nm, ou
lorsque le microbe est une bactérie, les nanoparticules métalliques ont une taille de particule de 7 nm à 12 nm, comme de 8 nm à 10 nm, ou
lorsque le microbe est un champignon, les nanoparticules métalliques ont une taille de particule de 10 nm à 18 nm, comme de 11 nm à 16 nm, ou de 12 nm à 15 nm, et
la composition antimicrobienne désactivant ou tuant le microbe.

6. Procédé selon la revendication 5, dans lequel la composition antimicrobienne est **caractérisée par** les caractéristiques d'une ou plusieurs des revendications 2 à 4.
